# EUROPEAN PATENT APPLICATION

(11) **EP 1 839 615 A1**
(43) Date of publication of application: **03.10.2007**
(21) Application number: 06711738.2
(22) Date of filing: 16.01.2006
(51) Int. Cl.: A61B 19/00, A61B 5/00

(54) **MEDICAL DEVICE DATA ANALYSIS DEVICE**

(30) Priority: 19.01.2005 JP 2005012090
(71) Applicant: Olympus Corporation, Tokyo 151-0072 (JP)
(72) Inventor: TASHIRO, Koichi c/o Olympus Medical Systems Corp., Tokyo 151-0072 (JP); UCHIKUBO, Akinobu c/o Olympus Medical Systems Corp., Tokyo 151-0072 (JP); GOTANDA, Masakazu c/o Olympus Medical Systems Corp., Tokyo 151-0072 (JP); IMAMIYA, Chie c/o Olympus Medical Systems Corp., Tokyo 151-0072 (JP); NAKAMURA, Takeaki c/o Olympus Medical Systems Corp., Tokyo 151-0072 (JP); OZAKI, Takashi c/o Olympus Medical Systems Corp., Tokyo 151-0072 (JP)
(74) Representative: von Hellfeld, Axel
(86) International application number: PCT/JP2006/300457
(87) International publication number: WO 2006/077798

(57) **Abstract**

The medical device data analysis device (57, 22) of the present invention are used for analyzing plural kinds of data of plural medical devices which have been obtained during an operation, and includes a data input portion for inputting the plural kinds of data outputted from the plural medical devices respectively, a data storage portion for storing the plural kinds of data outputted from the medical devices and inputted through the data input portion, and a display processing portion for processing the plural kinds of data stored in the data storage portion so as to be displayed, wherein the display processing portion executes a graph display processing for allowing display of the plural kinds of data stored in the data storage portion in a graph with a same time axis.

## Description

### Technical Field

The present invention relates to a medical device data analysis device for analyzing plural kinds of data with respect to medical devices obtained during the operation.

### Background Art

Recently the medical devices have been diversified into variety of use accompanied with development of the medical technology, showing the trend of improved functions. The medical device includes an electric cautery unit, an ultrasonic processor, and a surgical laser. Each of the aforementioned medical devices may be used independently. However, they may be combined to form a composite medical system.

Japanese Unexamined Patent Application Publication No. 2002-233535 discloses the operation system which includes a plurality of medical devices as the aforementioned medical system.

In the conventionally employed operation system, the respective medical devices are operated through the same communication interface and the same communication protocol such that those medical devices are centrally controlled by a system controller via the respective communication sections.

The aforementioned conventionally employed operation system stores plural kinds of data outputted from the respective medical devices, for example, data with respect to the operation devices such as image data of the endoscopic images, peritoneal cavity pressure data derived from the pneumoperitoneum unit, and ultrasonic output data outputted from the ultrasonic processor, and living body information derived from a patient monitor unit, for example, blood pressure, pulse and the like. After the operation, the physician reads the thus stored plural kinds of data of the medical devices, which are analyzed and processed to be displayed such that the medical treatment record is made.

Conventionally, the obtained data classified by the respective medical devices will be displayed in the tabular form with the time code during the analysis.

As the plural kinds of data outputted from the plural medical devices are represented in tables each having a different time axis, it is difficult to correlate those data. Therefore it may be difficult for the person who analyzes the data to confirm the content or condition of the operation.

The present invention has been made in consideration for the aforementioned points, and it is an object of the present invention to provide a medical device data analysis device capable of efficiently performing the data analysis.

### Disclosure of Invention

### Means for Solving the Problem

The present invention provides a medical device data analysis device for analyzing plural kinds of data with respect to plural medical devices which have been obtained during an operation, which includes a data input portion for inputting the plural kinds of data outputted from the plural medical devices respectively, a data storage portion for storing the plural kinds of data outputted from the medical devices and inputted through the data input portion, and a display processing portion for processing the plural kinds of data stored in the data storage portion so as to be displayed, wherein the display processing portion executes a graph display processing for allowing display of the plural kinds of data stored in the data storage portion in a graph with a same time axis.

### Brief Description of the Drawings

Fig. 1 is a view showing an entire structure of an operation support system of an embodiment.
Fig. 2 is a view showing an exemplary structure of the operation devices laid out in a first operation room.
Fig. 3 is a view showing a front panel of a pneumoperitoneum unit shown in Fig. 2.
Fig. 4 is a graph showing the peritoneal cavity pressure measured by the pneumoperitoneum unit shown in Fig. 3 with respect to the time axis.
Fig. 5 is a view schematically showing the structure of a computer in a conference room shown in Fig. 1 as the medical device data analysis device of the embodiment.
Fig. 6 is an exemplary display of a screen of the graph with the same time axis displayed on the monitor of the computer in the conference room shown in Fig. 5.
Fig. 7 is an exemplary display of a numerical value screen switched from the screen of the graph with the same time axis as shown in Fig. 6.
Fig. 8 is an exemplary display of the screen of the graph with the same time axis shown in Fig. 6 where the area designation is performed.
Fig. 9 is an exemplary display of a magnified display screen switched from the screen of the graph with the same time axis shown in Fig. 8.
Fig. 10 is an exemplary display/print screen on which the graph of the respective data displayed on the graph with the same time axis shown in Fig. 6 is selected so as to be displayed or printed.
Fig. 11 is a system structure view showing an example where the system controller is connected to the medical device which is not controlled thereby but data communicated therewith.
Fig. 12 is a view showing an exemplary screen for operating an electric cautery unit.
Fig. 13 is a view showing an exemplary screen which allows the operation or control of the pneumoperitoneum unit.
Fig. 14 is a view showing an exemplary alarm screen which notifies abnormality in the saturated blood oxygen level (SPO2) value.

### Best Mode for Carrying Out the Invention

Embodiments will be described hereinafter referring to the drawings.

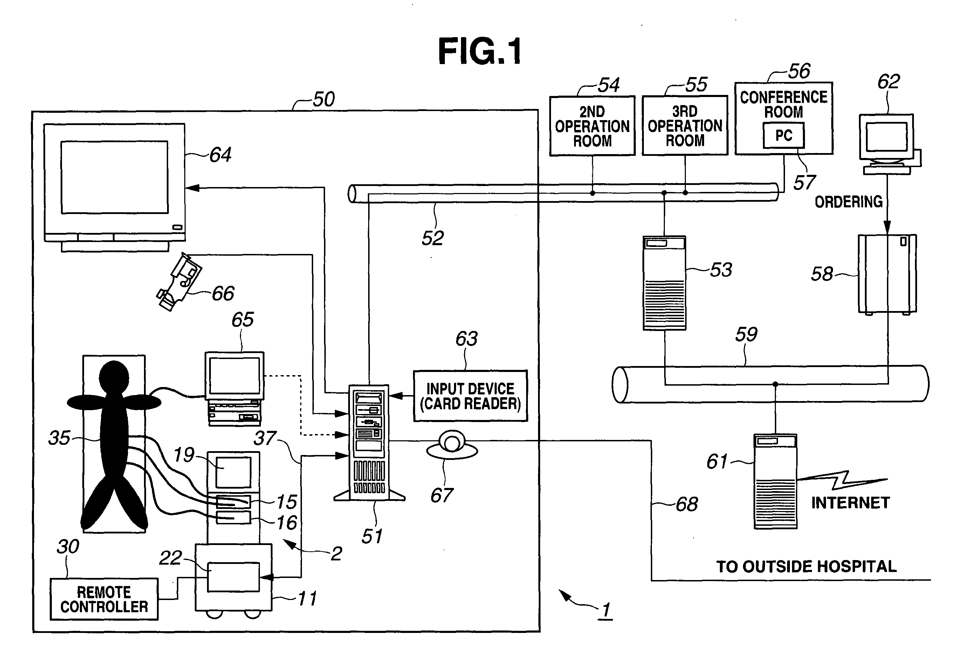

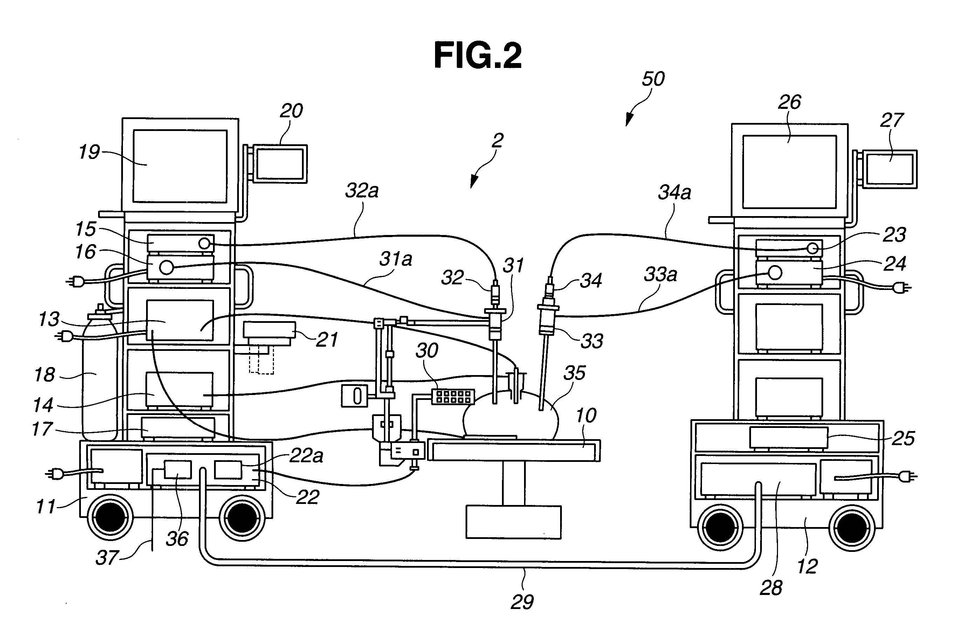

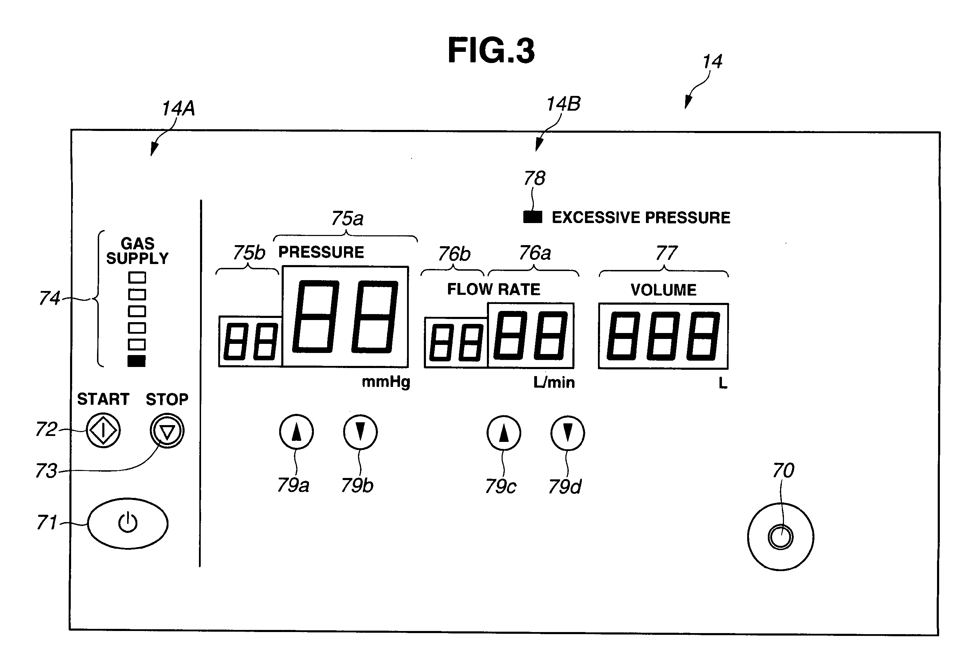

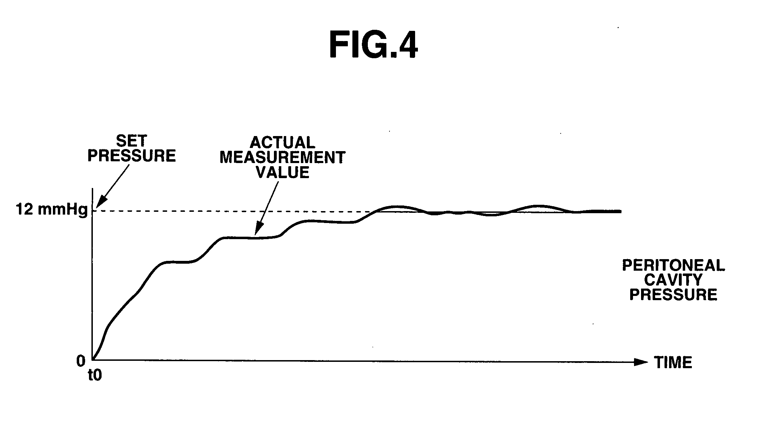

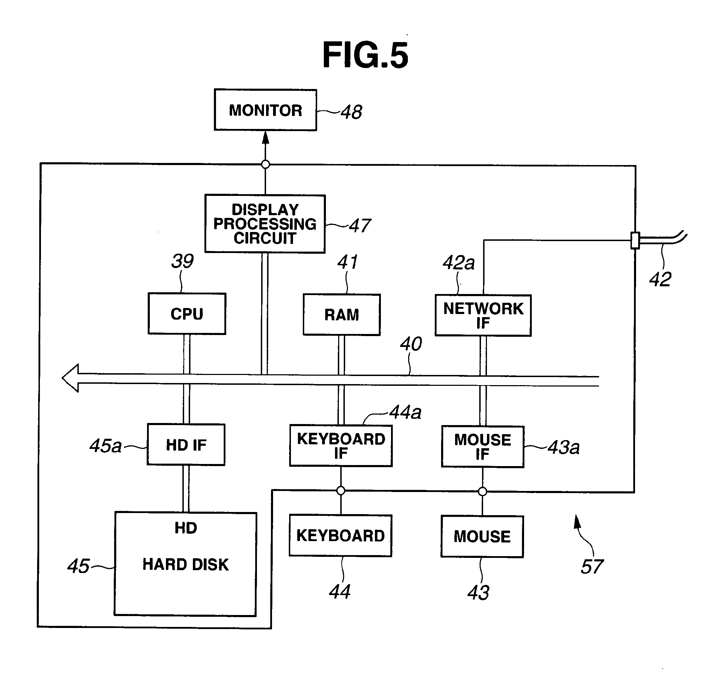

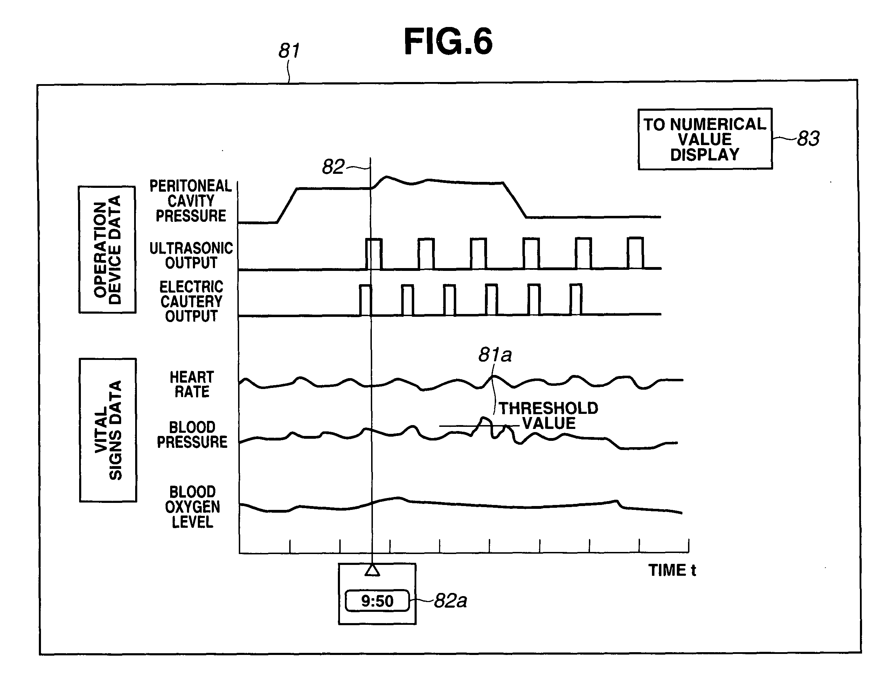

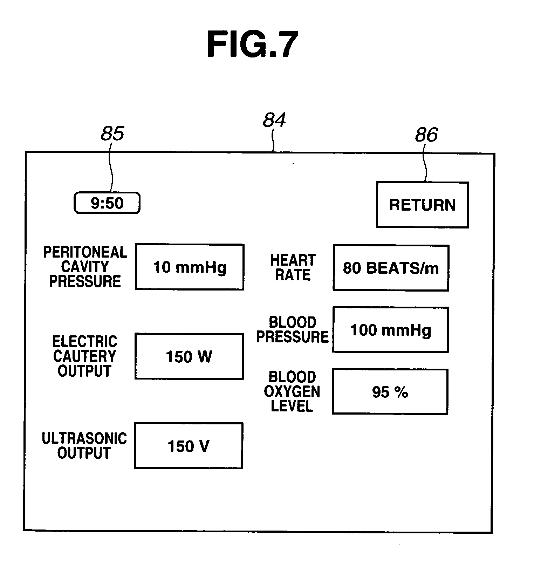

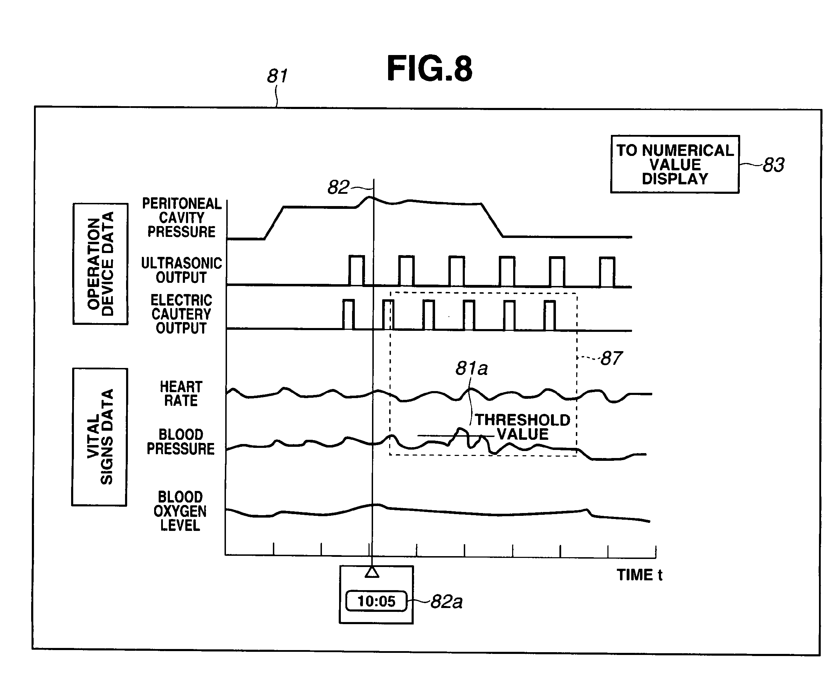

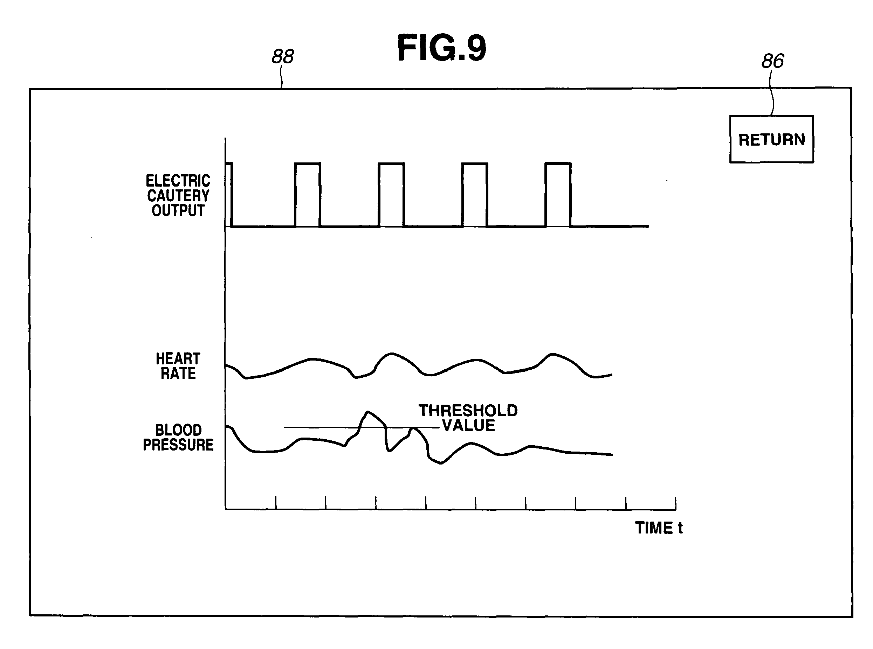

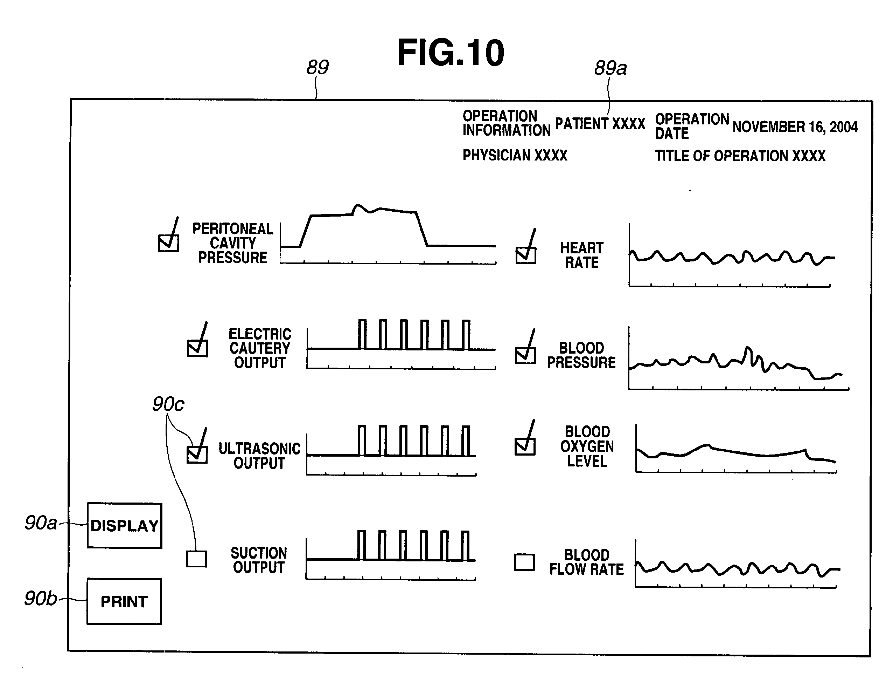

Figs. 1 to 10 show an embodiment. Fig. 1 is a view showing an entire structure of an operation support system of the embodiment. Fig. 2 is a view showing an exemplary structure of the operation devices laid out in a first operation room. Fig. 3 is a view showing a front panel of a pneumoperitoneum unit shown in Fig. 2. Fig. 4 is a graph showing the peritoneal cavity pressure measured by the pneumoperitoneum unit shown in Fig. 3 with respect to the time axis. Fig. 5 is a view schematically showing the structure of a computer in a conference room shown in Fig. 1 as the medical device data analysis device of the embodiment. Fig. 6 is an exemplary display of a screen of the graph with the same time axis displayed on the monitor of the computer in the conference room shown in Fig. 5. Fig. 7 is an exemplary display of a numerical value screen switched from the screen of the graph with the same time axis as shown in Fig. 6. Fig. 8 is an exemplary display of the screen of the graph with the same time axis where the area designation is performed. Fig. 9 is an exemplary display of a magnified display screen switched from the screen of the graph with the same time axis shown in Fig. 8. Fig. 10 is an exemplary display/print screen on which the graph of the respective data displayed on the graph with the same time axis shown in Fig. 6 is selected so as to be displayed or printed.

Referring to Fig. 1, an operation support system 1 according to the embodiment has an operation device 2 shown in Fig. 2 placed in a first operation room 50, for example. The operation device 2 is connected to a first controller 51 disposed in the first operation room 50. Fig. 1 partially shows the operation device 2, each structure of which will be described later.

The first controller 51 is connected to an operation room server 53 via a communication line 52 such as an operation section LAN as well as connected to not shown second and third controllers disposed in a second operation room 54 and a third operation room 55, respectively.

The operation room server 53 accumulates various medical information data obtained from controllers in the respective operation rooms so as to generate the medical treatment record information. The communication line 52 connected to the operation room server 53 is further connected to a personal computer (hereinafter referred to as a computer) 57 in a conference room 56 which serves to execute the teleconference function for observing the condition of the operation room and the medical treatment performed in the respective operation rooms, and instructing with respect to the medical treatment.

An in-hospital server 58 serving as means for accumulating patient information is placed in another location different from that of the operation room server 53. The in-hospital server 58 is connected to the operation room server 53 via a communication line 59, which uploads the medical treatment record information classified by the patient (case history information such as the medical condition and the medical procedure information such as the medication) derived from the operation room server 53 so as to be organized and integrated.

The operation room server 53 is further connected to a WEB server 61 via the in-hospital communication line 59. The in-hospital server 58 is connected to a patient registration terminal 62 to which the patient registration information is inputted. The in-hospital server 58 accumulates the patient registration information from the patient registration terminal 62, and the medical treatment record information such as the case history information, the medical procedure information and the medical image information relative to the respective patient.

The first controller 51 in the first operation room 50 is equipped with input devices (a keyboard, magnetic card reader and the like) 63 as input means through which the information which identifies a patient 35 subjected to the operation performed in the first operation room 50 is inputted, a display unit 64 which organizes image data such as the endoscopic image, the operation device information, the patient information, the thumbnail image, and physician's finding based on the output from the first controller 51, a living body monitor unit 65 which acquires the living body information such as the blood pressure, pulse of the patient 35 to be supplied to the first controller 51, and a room camera 66 connected to the first controller 51 for shooting the room condition.

In the case where the operation is performed through the dialogue between the physician in the operation room 50, 54 or 55 and the physician in the conference room 56 using the teleconference system, the room camera 66 is structured to control the camera angle based on the instruction of the physician in the conference room 56 with respect to the shooting position.

The first controller 51 is connected to a communication line 68 connected to the outside the hospital via a signal transmission unit 67 such that the video signal derived from the operation device 2 is transmitted to the outside the hospital, or the externally obtained video signal is received to be displayed on the display unit 64.

Fig. 2 shows the arrangement of the operation device 2 shown in Fig. 1.

Referring to Fig. 2, the operation device 2 using endoscopes is disposed around a bed 10 on which the patient 35 lies down in the first operation room 50. The operation device 2 includes a first cart 11 and a second cart 12.

The first cart 11 has medical devices to be controlled mounted thereon, for example, the operation devices such as an electric cautery unit 13, a pneumoperitoneum unit 14, and an ultrasonic processor (not shown), devices such as an endoscopic camera unit (camera control unit or CCU) 15, a light source device 16, and a video tape recorder (VTR) 17, and a gas tank 18 filled with carbon dioxide. The endoscopic camera unit 15 is connected to a first camera head 32 installed in a first endoscope 31 via a camera cable 32a. The light source device 16 is connected to a first endoscope 31 via a light guide cable 31a.

The first cart 11 further has a display unit 19, a first center display panel 20, and an operation panel 21 mounted thereon. The display unit 19 is a TV monitor, for example, to display the endoscopic image and the like.

The center display panel 20 is a display unit capable of selectively displaying any data obtained during the operation. The operation panel 21 is a center operation unit formed of a display section, for example, a liquid crystal display and a touch sensor integrally provided on the display section, which may be operated by the nursing staff in the non-sterilized area.

The first cart 11 further has a system controller 22 as a control unit mounted thereon. The system controller 22 is connected to the electric cautery unit 13, the pneumoperitoneum unit 14, the endoscopic camera unit 15, the light source device 16, and the VTR 17 via not shown corresponding communication lines, respectively. The system controller 22 includes a built-in communication controller 36, which is connected to the first controller 51 shown in Fig. 1 via a communication cable 37. The system controller 22 may be structured to have the same function as that of the first controller 51.

The system controller 22 includes a CPU 22a which executes a predetermined control program to control the devices subjected to the control, for example, the electric cautery unit 13. The CPU 22a and the control program form the medical device control section for controlling the medical devices.

Meanwhile, the second cart 12 has the devices to be controlled mounted thereon such as an endoscopic camera unit 23, a light source device 24, an image processor 25, a display unit 26 and a second center display panel 27.

The endoscopic camera unit 23 is connected to a second camera head 34 installed in a second endoscope 33 via a camera cable 34a. The light source device 24 is connected to the second endoscope 33 via a light guide cable 33a.

The display unit 26 displays the endoscopic image taken by the endoscopic camera unit 23 and the like. The second center display panel 27 is structured to be capable of selectively displaying any data obtained during the operation.

A relay unit 28 mounted on the second cart 12 is connected to the aforementioned endoscopic camera unit 23, the light source device 24 and the image processor 25 via not shown corresponding communication lines, respectively. The relay unit 28 is connected to the system controller 22 mounted on the aforementioned first cart 11 via a relay cable 29.

Thus, the system controller 22 is structured to centrally control the camera unit 23, the light source device 24 and the image processor 25 mounted on the second cart 12 and the electric cautery unit 13, the pneumoperitoneum unit 14, the camera unit 15, the light source device 16 and the VTR 17 mounted on the first cart 11.

Consequently, during the communication between the system controller 22 and the aforementioned devices, the system controller 22 is structured to display each set state of the connected devices, the set screen showing the operation switch and the like on the liquid crystal display of the operation panel 21. The system controller 22 allows touching of the desired operation switch on the operation panel 21 to operate the touch sensor of the predetermined area such that the operation input is performed for changing the set value and the like.

A remote controller 30 is a second center operation unit operated by a surgeon (operator) in the sterilized area, which allows other communicated devices to be operated through the system controller 22. The system controller 22 is capable of analyzing the living body information obtained through the first controller 51, and displaying the analysis results on the desired display unit.

In the case where the operation is performed with the operation device 2 in the first operation room 50, for example, the display units 19, 26 and/or the center display panels 20, 27 display image data shot by the first and the second camera heads 32, 34 installed in the endoscopes 31, 33 and subjected to the signal processing in the endoscopic camera units 15, 23, and plural kinds of data of the operation devices including the electric cautery unit 13, the pneumoperitoneum unit 14, the ultrasonic processor (not shown) and the like. The aforementioned image data and the plural kinds of data are transmitted substantially real time to the first controller 51 through the communication controller 36 so as to be accumulated in the memory unit such as the hard disk in the first controller 51. The memory unit like the hard disk in the first controller 51 further accumulates the living body information and the patient information derived from the living body monitor unit 65.

After the operation, the computer 57 in the conference room 56 uploads the image data of the endoscopic images, the plural kinds of data with respect to the operation devices, the living body information and the patient information accumulated in the first controller 51 to be stored in the hard disk in the computer 57, and allows the stored data to be edited, for example, for the analysis and display.

In the case where the operation in the first operation room 50 is supported from the conference room 56, the monitor 48 (see Fig. 5) of the computer 57 in the conference room 56 is allowed to display the image data of the endoscopic images shot during the operation in the first operation room 50 so as to give the support or advice. The computer 57 is not limited to the thus set terminal, but may be a portable device such as a PDA.

The pneumoperitoneum unit 14 as an example of the operation device will be described.

Referring to Fig. 3, the front panel of the pneumoperitoneum unit 14 is divided into a source set display section 14A for setting, operating and displaying data with respect to the gas tank 18, and a peritoneal cavity set display section 14B for setting, operating and displaying data with respect to the peritoneal cavity. A peritoneal cavity supply cap 70 serving as the air supply port for the peritoneal cavity is provided at the lower portion of the peritoneal cavity set display section 14B.

The source set display section 14A is provided with a power switch 71, an air supply start button 72, an air supply stop button 73 and a gas supply level display section 74. The peritoneal cavity set display section 14B is provided with a peritoneal cavity pressure display section 75a, a peritoneal cavity pressure set display section 75b, a peritoneal cavity flow rate display section 76a, a peritoneal cavity flow rate set display section 76b, a supply total gas volume display section 77, a pressure alarm lamp 78, peritoneal cavity pressure set buttons 79a, 79b, and supply gas flow rate set buttons 79c, 79d.

The power switch 71 is operated for switching the power source of the pneumoperitoneum unit 14 between ON and OFF. The pneumoperitoneum unit may be made operable by turning the power switch 71 ON. The air supply start button 72 is operated to command to start the gas supply to the peritoneal cavity. The air supply stop button 73 is operated to command to stop the gas supply to the peritoneal cavity.

The peritoneal cavity pressure set button 79a and the supply gas flow rate set button 79c are operated to gradually increase the set value. Meanwhile, the peritoneal cavity pressure set button 79b and the supply gas flow rate set button 79d are operated to gradually decrease the set value.

The gas supply level display section 74 displays the amount of gas supplied from the gas tank 18. The peritoneal cavity pressure display section 75a displays the measurement result of the peritoneal cavity pressure measured by the pressure sensor. Meanwhile, the peritoneal cavity pressure set display section 75b displays the pressure set value of the peritoneal cavity pressure set by operating, for example, the peritoneal cavity pressure set buttons 79a and 79b.

The peritoneal cavity flow rate display section 76a displays the measurement result of the peritoneal cavity flow rate measured by the flow sensor. Meanwhile, the peritoneal cavity flow rate set display section 76b displays the flow rate set value of the peritoneal cavity flow rate set by operating the supply gas flow rate set buttons 79c and 79d. The supply total gas volume display section 77 displays the total volume of supplied gas calculated based on the measurement value of the flow sensor. The pressure alarm lamp 78 is switched from the OFF state to the flashing display state or the red illuminated state to alarm the operator and the like that the peritoneal cavity pressure has become higher than the set value.

The peritoneal cavity pressure, flow rate of the supplied gas to the peritoneal cavity may be set on the operation panel 21. Alternatively, the center display panel 20 may be structured to display one or a plurality of preliminarily designated values from those displayed on the peritoneal cavity pressure display section 75a, the peritoneal cavity pressure set display section 75b, the peritoneal cavity flow rate display section 76a, the peritoneal cavity flow rate set display section 76b, and the supply total gas volume display section 77.

The peritoneal cavity pressure of the patient is controlled as shown in Fig. 4 by the use of the thus structured pneumoperitoneum unit 14. In the example, 12 mmHg is set as the peritoneal cavity pressure set value.

The computer 57 in the conference room 56 may be used for supporting the operation performed in the first operation room 50, and reading the image data such as the endoscopic images, the plural kinds of data with respect to the operation devices, the living body information and the patient information which are accumulated in the first controller 51 so as to be stored in the hard disk of the computer 57 to edit the stored data for the analysis, display and the like.

The computer 57 exhibits functions of the medical device data analysis device according to the embodiment as shown by its main components in Fig. 5.

In the computer 57, a CPU 39 for executing the entire control is connected to an internal bus 40. The internal bus 40 is connected to a RAM 41 used for the work area of the CPU 39, a network interface 42a connected to a cable 42 linked to the communication line 52, and a mouse interface 43a connected to a mouse 43 via a connector portion.

The internal bus 40 is connected to a keyboard 44 and a hard disk 45 (abbreviated to HD in Fig. 5) which stores the program and the image data via the corresponding interfaces 44a and 45a. The internal bus 40 is further connected to a monitor 48 which displays the image via a display processing circuit 47 for performing the display processing.

The CPU 39 reads the program installed in the hard disk 45 first so as to be written into a predetermined area within the RAM 41 such that the program is executed thereafter. The hard disk 45 is used for storing the image data such as the endoscopic images, the plural kinds of data with respect to the operation devices including the electric cautery unit 13, the pneumoperitoneum unit 14, the ultrasonic processor (not shown), the living body information and the patient information from the living body monitor unit 65.

The hard disk 45 serving as the memory unit exhibits the function of the data storage unit for storing the plural kinds of data from the medical devices which have been obtained during the operation.

For example, the surgeon (operator) who performs the operation in the first operation room 50 operates the keyboard 44 after the operation to input the command to the CPU 39 so as to upload the image data such as the endoscopic image of the patient 52 stored in the first controller 51, plural kinds of data of the operation devices including the electric cautery unit 13, the pneumoperitoneum unit 14, the ultrasonic processor (not shown), and the living body information and the patient information from the living body monitor unit 65 to the hard disk 45 via the network interface 42a. The network interface 42a connected to the cable 42 forms the data input section for inputting the plural kinds of data outputted from the corresponding medical devices. The CPU 39 for writing the data into the hard disk 45, the program for writing the data, and the interface 45a form the data storage section for storing the plural kinds of data inputted from the data input section into the hard disk 45 as the memory unit.

The operator edits the data stored in the hard disk 45 to form the file adapted to the use for the diagnosis and subsequent operations. As occasion demands, the other required image data may be read from the first controller 51 and the operation room server 53 so as to be used.

The monitor 48 of the computer 57 may be structured to display the video taken during the operation (live video of the operation) such that the operation is supported from the conference room 56. If the setting to command storage of the video image data is performed through the keyboard 44, the image data may be stored in the hard disk 45.

In the embodiment, the plural kinds of data stored in the hard disk 45 are processed to be displayed in the form of the graph with respect to the same time axis for the analysis after the operation.

That is, the display processing circuit 47 processes the plural kinds of data of the operation devices and the living body information stored in the hard disk 45 to be displayed in the form of the graph with respect to the same time axis such that the screen of the graph with the same time axis may be displayed on the monitor 48. The CPU 39 of the computer 57 and the program for displaying the respective views shown in Figs. 6 to 10 to be described later form the display processing section for processing the plural kinds of data stored in the hard disk 45 to be displayed.

Referring to Fig. 6, a display screen 81 of the graph with the same time axis shows the operation device data and the vital signs data as the living body information with respect to the same time axis.

In Fig. 6, the data shown as the operation device data include the peritoneal cavity pressure data derived from the pneumoperitoneum unit 14, the ultrasonic output data outputted from the ultrasonic processor (not shown), and electric cautery output data outputted from the electric cautery unit 13. Meanwhile, the data shown as the vital signs data include those of heart rate, blood pressure, blood oxygen level and the like. If each of the respective data exceeds the predetermined threshold value, the subject threshold value may be displayed on the graph of the "blood pressure" as shown by a threshold value display 81 a.

The operator is allowed to confirm the plural data of the medical devices with the same time axis by observing the display screen 81 of the graph with the same time axis. Accordingly, the respective data may be easily correlated with one another, allowing easy confirmation with respect to the operation content and the operation condition.

There is a time designation bar 82 which is horizontally movable in the left-to-right direction on the graph along the time axis in response to the clicking operation of the mouse 43 on the display screen 81 of the graph with the same time axis. A time display section 82a is provided below the time designation bar 82. The time display section 82a horizontally moves in the left-to-right direction along the time axis together with the time designation bar 82 so as to display the time corresponding to the position of the bar.

When a "numerical value display" button 83 is clicked through the mouse 43, the display screen 81 of the graph with the same time axis is switched to a numerical value display screen which shows the respective data values at the time corresponding to the position of the time designation bar 82.

Referring to Fig. 7, the numerical value display screen 84 shows the time designated by the time designation bar 82 in a time display box 85 as well as the respective values of the operation device data and the vital signs data as the living body information at the displayed time. For example, "10 mmHg" is displayed as the peritoneal cavity pressure, "150 W" is displayed as the electric cautery output value, and "100 mmHg" is displayed as the blood pressure value at the time "9:50".

The operator is allowed to confirm the plural data of the medical devices at the desired time in further detail while observing the numerical value display screen 84.

The numerical value display screen 84 may be returned to the display screen 81 of the graph with the same time axis shown in Fig. 6 by clicking a "return" button 86 through the mouse 43.

On the display screen 81 of the graph with the same time axis shown in Fig. 6, the operator is allowed to designate the desired area, for example, the area around the threshold value display 81 a as shown in Fig. 8 by clicking and dragging the mouse 43 on the graph display section.

Then the display screen 81 of the graph with the same time axis is switched to a magnified display screen 88 on which the time width is magnified with respect to the designated area (area enclosed by the dashed line in Fig. 8) 87.

The magnified display screen 88 shown in Fig. 9 displays the thus cut designated area 87 shown in Fig. 8 on which the time width has been magnified. The area may be magnified with the time width determined based on a preliminarily set magnification. However, the operator is allowed to arbitrarily set the magnification of the time width.

The operator is capable of confirming the correlation among the data in further detail with respect to the desired area while observing the magnified display screen 88 shown in Fig. 9.

The magnified display screen 88 is returned to the display screen 81 of the graph with the same time axis shown in Fig. 6 by clicking the "return" button 86 through the mouse 43. The designated area on the display screen 81 of the graph with the same time axis shown in Fig. 8 may be cut and copied so as to be pasted onto the medical treatment record.

Each graph of the respective data to be displayed on the display screen 81 of the graph with the same time axis may be selected on the display/print screen so as to be displayed or printed.

Referring to Fig. 10, the display/print screen 89 displays the operation information section 89a on which the operation data such as the name of the patient, the operation date are displayed at the upper portion. All the graphs of the respective data each having the different time width are displayed below the operation information section 89a. On the display/print screen 89, a display preset button 90a and a print preset button 90b are provided.

A check box 90c of the graph selected from those graphs each having the different time width is checked and the display preset button 90a is operated on the display/print screen 89 so as to be switched to the display screen 81 of the graph with the same time axis as described referring to Fig. 6. The selected graphs of the data are all displayed with the same time axis. The display/print screen 89 and the processing which sets the data corresponding to the checked check box 90c as the data to be displayed form the set section for setting the data from the inputted plural kinds of data to be displayed.

The check box 90c on the display/print screen 89 forms the display selection section which allows selection of the data to be displayed from the plural kinds of data which have been displayed. The data to be displayed may be determined based on the determination whether or not the corresponding check box 90c has been checked.

Meanwhile, the check box 90c corresponding to one of the graphs each having the different time width is checked and the print preset button 90b is operated on the display/print screen 89 so as to print the selected graph of data. Accordingly, the display/print screen 89 and the processing which sets the data corresponding to the checked check box 90c as the data to be printed form the set section for setting the data from the inputted plural kinds of data to be printed.

The check box 90c on the display/print screen 89 forms the print selection section which allows selection of the data to be printed from the plural kinds of data which have been displayed. The data to be printed may be determined based on the determination whether or not the corresponding check box 90c has been checked.

The operator is allowed to preset the display or print on the basis of the same time axis by selecting the graph of the respective data, thus improving the operability.

In the embodiment, the plural kinds of data of the medical devices may be displayed on a single graph with the same time axis. The plural kinds of data of the medical devices may be represented by the plural graphs individually. Alternatively the data may be represented by the corresponding graphs each with the same time axis.

As a result, according to the embodiment, the stored plural kinds of data with respect to the medical devices may be displayed with the same time axis. This makes it possible to easily correlate those data as well as to easily confirm the operation content or the operation condition.

The system controller 22 controls the medical devices such as the endoscopic camera unit 15. The computer 57 executes the aforementioned processing upon reception of the respective data from the system controller 22 so as to display the plural kinds of data with the same time axis.

The functions of the screen display and the like shown in Figs. 6 to 10 are realized by the computer 57 in the embodiment. However those functions may be realized by the system controller 22 or the first controller 51. Assuming that the system controller 22 performs the aforementioned function of the computer 57, the CPU 22a, the not shown interface and the like form the data input section for inputting the plural kinds of data, the data storage section for storing the data, the display processing section for displaying the screen as shown in Fig. 6 and the like.

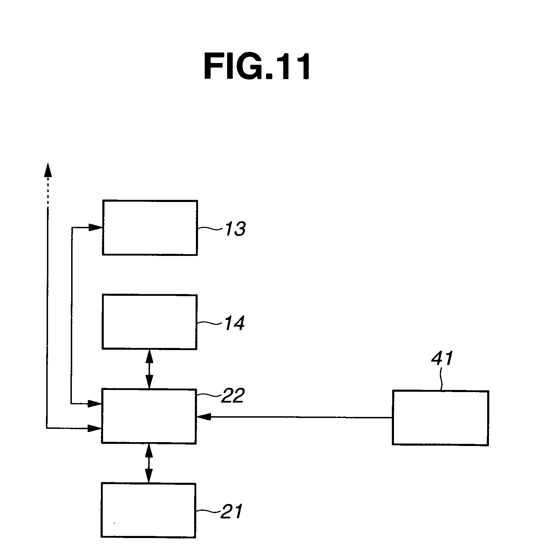

As described above, the system controller 22 is connected to the respective medical devices for performing the data communication and control. There may be the case where the system controller 22 is connected to the medical device not to be controlled thereby but data communicated therewith. Fig. 11 shows the system structure representing that the system controller 22 is connected to the medical device not to be controlled thereby but data communicated therewith. Referring to Fig. 11, the same components as those of the structure shown in Fig. 2 will be designated with the same codes. The explanations of those components, thus, will be omitted. The structure shown in Fig. 11 is substantially the same as that shown in Fig. 2 except that an anesthesia unit 41 connected to the system controller 22 as a medical device which is only data communicated therewith is added to the structure shown in Fig. 2. The other medical devices unrelated to the following explanation are not shown in Fig. 11.

In the surgery with the endoscope, the anesthesia unit 41 is employed in addition to the electric cautery unit 13 and the pneumoperitoneum unit 14. During the surgery with the endoscope, the pneumoperitoneum unit 14 is used for supplying carbon dioxide gas into the peritoneal cavity of the patient such that the operator has sufficient visual field and working space.

The anesthesia unit 41 holds the data such as the anesthesia record information and the saturated blood oxygen level (SPO2). The system controller 22 controls and communicates with the electric cautery unit 13 and the pneumoperitoneum unit 14 while communicating with the anesthesia unit 41. In the aforementioned state, if it is determined that the data value of the saturated blood oxygen level (SPO2) received from the anesthesia unit 41 indicates the abnormality compared with the predetermined value, the system controller 22 allows the physician and the nursing staff to operate the pneumoperitoneum unit 14.

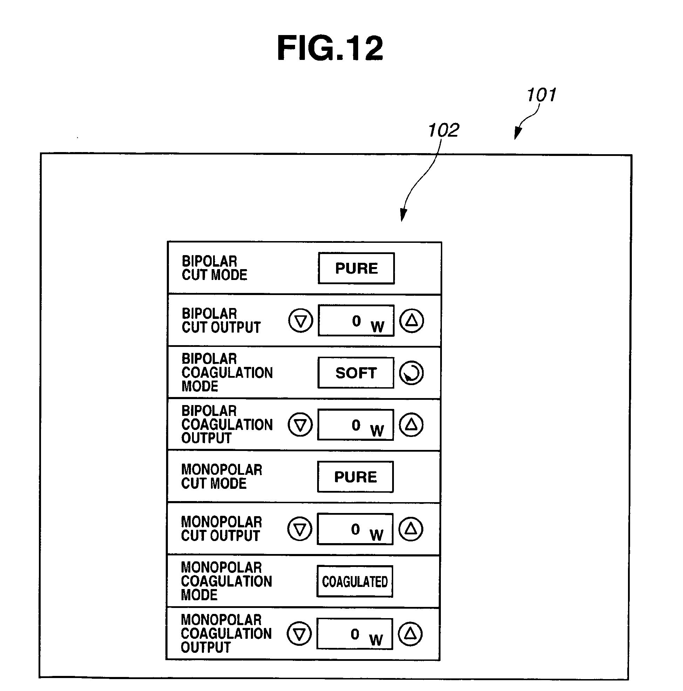

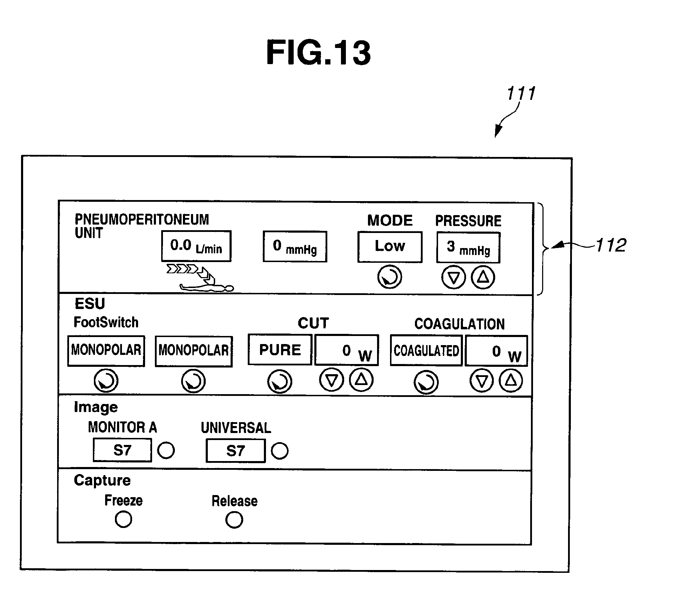

For example, if the value of the saturated blood oxygen level (SPO2) is determined as being abnormal on the operation screen of the electric cautery unit 13 as shown in Fig. 12, the screen which allows the operation of the pneumoperitoneum unit 14 as shown in Fig. 13 is displayed on the display section of the operation panel 21. Fig. 12 is the view showing an example of the operation screen of the electric cautery unit 13. Fig. 13 is a view showing an example of the screen which allows the operation or control of the pneumoperitoneum unit 14. An operation screen 101 shown in Fig. 12 includes a display section 102 which relates to various outputs of the electric cautery unit 13. An operation screen 111 shown in Fig. 13 includes a display section 112 for the various operation or control of the pneumoperitoneum unit 14. The display section 112 displays such states as the flow rate, pressure, mode of the pneumoperitoneum unit 14, and includes an operation button for setting the mode and the pressure.

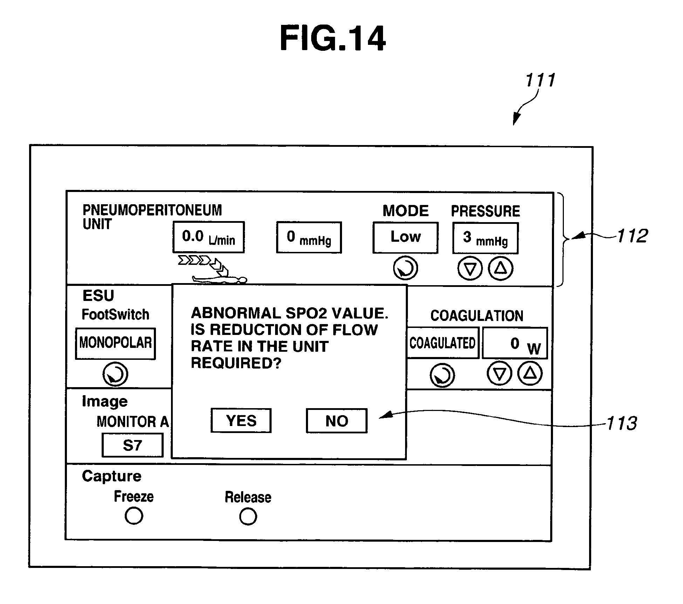

In the aforementioned case, an alarm screen for notifying the physician of the abnormality in the value of the saturated blood oxygen level (SPO2) may be displayed on the display unit. Fig. 14 is a view showing an example of the alarm screen for notifying the abnormality in the value of the saturated blood oxygen level (SP02). An alarm screen 113 is displayed on a pop-up window of the display on the operation screen 111 shown in Fig. 13. The alarm screen 113 may include an abnormal content display section for displaying the content of the abnormality through the message "the SPO2 value is abnormal.", an operation content display section for displaying the operation content required for coping with the abnormality through the message "Is reduction in the flow rate of the unit required ?", and an operation command section for commanding the operation through selection between "YES" and "NO" so as to allow the physician and the nursing staff to perform various operations after confirming the abnormality content.

The aforementioned structures may be partially combined to form the embodiment according to the present invention.

### Industrial Applicability

According to the present invention, the medical device data analysis device allows easy correlation among stored plural kinds of data with respect to the medical devices, and is suitable for the data analysis to form the medical treatment records or the like.

## Claims

1. A medical device data analysis device for analyzing plural kinds of data with respect to plural medical devices which have been obtained during an operation, comprising:
a data input portion for inputting the plural kinds of data outputted from the plural medical devices respectively;
a data storage portion for storing the plural kinds of data inputted through the data input portion; and
a display processing portion for processing the plural kinds of data stored in the data storage portion so as to be displayed, wherein the display processing portion executes a graph display processing for allowing display of the plural kinds of data stored in the data storage portion in a graph with a same time axis.

2. The medical device data analysis device according to Claim 1, further comprising a medical device control portion for controlling the plural medical devices.

3. The medical device data analysis device according to Claim 1 or 2, wherein:
the plural medical devices include an operation device or a living body monitor unit used for the operation;
the data storage portion stores operation data outputted from the operation device and living body information outputted from the living body monitor unit; and
the display processing portion executes the graph display processing for allowing display of the operation data and the living body information in the graph with the same time axis.

4. The medical device data analysis device according to Claim 1 or 2, wherein the display processing portion executes a display processing to display data values at a designated time on the graph displayed with the same time axis.

5. The medical device data analysis device according to Claim 1 or 2, wherein the display processing portion executes a magnified display processing for magnifying to display a designated area of the graph displayed with the same time axis.

6. The medical device data analysis device according to Claim 1 or 2, further comprising a set portion for setting data to be displayed from the plural kinds of data inputted through the data input portion.

7. The medical device data analysis device according to Claim 6, wherein the set portion includes a display selection portion which allows the plural kinds of data inputted through the data input portion to be displayed on a display unit and allows the data to be displayed to be selected from the displayed plural kinds of data such that the data to be displayed are set.

8. The medical device data analysis device according to Claim 1, wherein the data input portion inputs the plural kinds of data obtained from a medical device control portion for controlling the plural medical devices.

9. The medical device data analysis device according to Claim 8, wherein:
the plural medical devices include an operation device or a living body monitor unit used for the operation;
the data storage portion stores operation data outputted from the operation device and living body information outputted from the living body monitor unit; and
the display processing portion executes the graph display processing for allowing display of the operation data and the living body information in the graph with the same time axis.

10. The medical device data analysis device according to Claim 8, wherein the display processing portion executes a display processing to display data values at a designated time on the graph displayed with the same time axis.

11. The medical device data analysis device according to Claim 8, wherein the display processing portion executes a magnified display processing for magnifying to display a designated area of the graph displayed with the same time axis.

12. The medical device data analysis device according to Claim 8, further comprising a set portion for setting data to be displayed from the plural kinds of data inputted through the data input portion.

13. The medical device data analysis device according to Claim 12, wherein the set portion includes a display selection portion which allows the plural kinds of data inputted through the data input portion to be displayed on a display unit and allows the data to be displayed to be selected from the displayed plural kinds of data such that the data to be displayed are set.
